Europäisches Patentamt

European Patent Office

Office européen des brevets

(19)

(11) Publication number: **0 249 053 B1**

(12) **EUROPEAN PATENT SPECIFICATION**

(45) Date of publication of patent specification: **29.01.92** (51) Int. Cl.⁵: **C07C 43/20**, C07C 41/14

(21) Application number: **87107094.2**

(22) Date of filing: **15.05.87**

---

(54) Process for the preparation of alkylaryl ethers.

---

(30) Priority: **27.05.86 IT 2057486**

(43) Date of publication of application:
**16.12.87 Bulletin 87/51**

(45) Publication of the grant of the patent:
**29.01.92 Bulletin 92/05**

(84) Designated Contracting States:
**AT BE CH DE ES FR GB GR IT LI LU NL SE**

(56) References cited:
**EP-A- 0 196 687
DE-C- 827 803
FR-A- 2 149 461**

**JOURNAL OF THE CHEMICAL SOCIETY, part II, 1947, pages 928-935, The Chemical Society, London, GB; P.H. GIVEN et al.: "Some catalysed gas-phase reactions of aromatic hydrocarbons. Part I. The interaction of benzene with methyl ether"**

(73) Proprietor: **ENIRICERCHE S.p.A.
Corso Venezia 16
I-20121 Milan(IT)**

(72) Inventor: **Brunelli, Maurizio
Via Agadir, 10/C
San Donato Milanese (Milano)(IT)**
Inventor: **Bellussi, Giuseppe
Via Alberto Scoto, 44
Piacenza(IT)**

(74) Representative: **Cioni, Carlo et al
c/o ENIRICERCHE S.p.A. BRELID Via F. Maritano 26
I-20097 San Donato Milanese(IT)**

---

Rank Xerox (UK) Business Services

**Description**

The present invention relates to a process for the preparation of alkylaryl ethers.

More particularly the present invention relates to a process for the preparation of alkylaryl ethers starting from phenols and ethers of aliphatic alcohols.

It is known the preparation of alkylaryl ethers starting from phenols and ethers of aliphatic alcohols in the presence of strongly acidic ion exchange resins (European Patent 13924).

The use of ion exchange resins has, however, the drawback that it is not possible to operate at high temperatures for long periods because said resins tend to lose at said conditions, their physical and mechanical characteristics.

Accordingly either it is necessary to operate at low temperature with the consequent strong lowering of the yields or, by working at high temperature, the resin needs to be frequently replaced.

On the contrary it is known from German Patent 827803 a method for preparing methyl ether or methanol with phenols in the presence of $BPO_4$, which operates at high temperature with high yelds.

In particular in the example 1 of this patent methanol is reacted with pyrocatechol in the presence of $BPO_4$ at a temperature of $250°C$: the dimethyl ether is not added but it is obtained by the reaction and then it has gone out.

It has been surprisingly found and this is the object of the present invention that it is possible as regards the method claimed in DE-827803 to reduce considerably the reaction time, on obtaining the same conversion and the same selectivity, by the use of a catalytic system constituted by an amorphous $BPO_4$ in amounts by weight in the range of from 0,5 to 50% based on the weight on the reagents and by the use as reagent of the dimethyl ether and not methanol.

The process according to the present invention comprises the steps of contacting a phenol or a phenols mixture with an ether of aliphatic alcohols or a mixture of ethers of aliphatic alcohols, at a temperature of from 150 to $400°C$, preferably from 200 to $300°C$, in the presence of amorphous boron phosphate in amount by weight in the range of from 0,5 to 50% based on the weight of the reagents which has been calcined at a temperature of from 150 to $600°C$. The alkylaryl ether obtained then are separated from the other reaction components, by fractionation or crystallization.

A method for preparing boron phosphate, which method has not to be considered as restrictive of the invention, is disclosed in example 1.

The reaction between phenol or phenols mixture and either the ether of aliphatic alcohols or the mixture of ethers of aliphatic alcohols can occurr in the presence of a solvent or in the absence of the same.

As solvents, when they are used, use in made of hydrocarbons, preferably aromatic hydrocarbons, particularly toluene.

Boron phosphate is utilized in amounts by weight in the range of from 0,5% to 50% based on the weight of the reagents.

The process is carried out at atmospheric pressure or higher than the atmospheric pressure.

The process can be batch or continuous.

In the case of a continuous operation, the catalyst is placed in a fixed or fluid bed and the reagents are continuously fed through the same, while the reaction products are continuously withdrawn from the side opposite to that where the reagents are introduced.

In the case of a batch operation the catalyst at the end of the reaction is removed by filtration or centrifugation, before separating the alkylaryl ether.

Phenols can be chosen among: phenol, substituted phenols having $C_1$-$C_4$ alkyl groups, such as meta-, ortho-, para-cresol, xylenols and tert-butyl phenol, phenols substituted by halogen atoms (e.g. ortho-, metha-, para-chlorophenol), polyphenols such as hydroquinone and pyrocatechol, alpha- and beta-naphtol and hydroxyanthracene.

The ethers are chosen among the ethers of aliphatic alcohols having a number of carbon atoms in the range of from 1 to 8, preferably from 1 to 4, particularly dimethyl ether, diethyl ether, dipropyl ether, diisopropyl ether, diisobutyl ether and di tert-butil ether.

Some examples will now be provided with the aim of a better illustration of the invention , being understood that the same has not be considered restricted to them or by them.

Example No.1

In a pyrex glass beaker 1000g distilled water were heated to $70°C$ and there were added to them, under stirring, 92.7 g $H_3BO_3$.

When all $H_3BO_3$ was dissolved, always under stirring, there were added g 172.8 $H_3PO_4$ (85% aqueous

solution).

Stirring was continued and water evaporated until a slurry was obtained.

The solid was dried at 100°C and a portion thereof was calcined at 170°C and another portion thereof was calcined at 550°C for two hours.

The calcined product was then washed 4 times, redispersing the same each time in one litre of distilled water kept boiling, filtering and drying.

Example No.2

Into a 40 ml microreactor, heated by a sand bath and provided with an external system of mechanical stirring, there were charged 10.0 g of amorphous BPO₄, prepared as described in Example 1 and calcined at 550°C, 10.0 g of hydroquinone and 14.8 g of dimethyl ether at a weight ratio 1/1/1.48.

The reagents were kept for two hours at the temperature of 280°C. The hydroquinone conversion was 44% by moles, the yield to methyl hydroquinone 33% by moles and the selectivity to methyl hydroquinone 75% by moles.

The gascromatographic amolysis of the obtained products gave the following results:

```
methyl hydroquinone:    34.3 % by weight

dimethyl hydroquinone: 13.3 % "        "

hydroquinone         : 52.4 % "        "
```

Example No.3

In the same microreactor with the previous procedures there were charged 8.0 g amorphous BPO₄ calcined at 170°C, 8.0 g hydroquinone and 7.9 g dimethyl ether, at a weight ratio 1/1/1. The hydroquinone conversion was 52% by moles, the yield to methyl hydroquinone 45% by moles and the selectivity to methyl hydroquinone 87% by moles.

The gas-chromatographic analysis of the products gave the following result:

```
methyl hydroquinone   : 47.1 % by weight

dimethyl hydroquinone : 7.6 % "        "

hydroquinone          : 45.3 % "        "
```

Example No.4

Into a microreactor, heated by a sand bath and provided with a mechanical stirring system there were charged 10.3 g of amorphous BPO₄ prepared as in example 1 and calcined at 550°C, 10.2 g pyrocatechol and 10,3 g dimethyl ether at a weight ratio 1/1/1.3.

The reagents were kept for two hours at the temperature of 280°C.

The pyrocatechol conversion was 40%, the yield to guaiacol 37% and the selectivity to guaiacol 92%. The gas-chromatographyc analysis of the obtained products gave the following result:

```
guaiacol    : 39.6 % by weight

veratrole   : 3.4 % "        "

pyrocatechol : 57 % "        "
```

**Claims**

1. A process for preparing alkylarylethers which comprises reacting a phenol or a mixture of phenols with an ether of aliphatic alcohol or a mixture of ethers of aliphatic alcohols in presence of a catalyst system constituted by amorphous BPO$_4$ at a reaction temperature of from 200 to 300°C characterized in that the amorphous BPO$_4$, which has been calcined at a temperature of from 150 to 600°C, is utilized in amounts by weight in the range of from 0,5 to 50% based on the weight of the reagents.

**Revendications**

1. Procédé de préparation d'alkyl-aryl-éthers, qui comporte la réaction d'un phénol ou d'un mélange de phénols avec un éther d'alcool aliphatique ou un mélange d'éthers d'alcools aliphatiques, en présence d'un système catalytique constitué par du BPO$_4$ amorphe, à une température de réaction valant de 200 à 300°C, caractérisé en ce que le BPO$_4$ amorphe, qui a été calciné à une température valant de 150 à 600°C, est utilisé en un poids valant de 0,5% à 50% du poids des réactifs.

**Patentansprüche**

1. Verfahren zur Herstellung von Alkylarylethern, welches ein Umsetzen eines Phenols oder eines Gemisches von Phenolen mit einem Ether eines aliphatischen Alkohols oder einem Gemisch von Ethern von aliphatischen Alkoholen in Gegenwart eines aus amorphem BPO$_4$ bestehenden Katalysatorsystems bei einer Reaktionstemperatur von 200 bis 300°C umfaßt, dadurch gekennzeichnet, daß das amorphe BPO$_4$, das bei einer Temperatur von 150 bis 600°C calciniert worden ist, in Gewichtsmengen im Bereich von 0,5 bis 50 %, bezogen auf das Gewicht der Reagentien, eingesetzt wird.